# EUROPÄISCHE PATENTANMELDUNG

(11) **EP 2 749 278 A1**
(43) Veröffentlichungstag der Anmeldung: **02.07.2014**
(21) Anmeldenummer: 14000779.0
(22) Anmeldetag: 26.05.2007
(51) Int. Cl.: A61K 31/155, A61K 31/4439, A61K 31/57, A61P 5/24, A61P 5/28, A61K 31/567, A61K 31/585

(54) **Kombination aus einem Gestagen und einem Insulinsensitizer zur Behandlung von PCOS**

(30) Priorität: 01.06.2006 DE 102006026026
(62) Teilanmeldung aus: 07725596.6
(71) Anmelder: Richter Gedeon NYRT, 1103 Budapest (HU)
(72) Erfinder: Schramm, Georg, 52224 Stolberg (DE)
(74) Vertreter: Kutzenberger, Helga

(57) **Zusammenfassung**

Die vorliegende Erfindung betrifft ein Arzneimittel, das als Wirkstoffkombination wenigstens ein kontrazeptiv wirkendes Gestagen als einzige Hormonkomponente und wenigstens einen Insulinsensitizer enthält, das sich insbesondere zur Prophylaxe gegen oder zur Therapierung von krankhaften Störungen, die durch das Polyzystische Ovar-Syndrom (PCOS) verursacht werden, eignet.

## Beschreibung

Die vorliegende Erfindung betrifft ein Arzneimittel, das als Wirkstoffkombination wenigstens ein kontrazeptiv wirkendes Gestagen als einzige Hormonkomponente und wenigstens einen Insulinsensitizer enthält, das sich insbesondere zur Prophylaxe gegen oder zur Therepierung von krankhaften Störungen, die durch das Polyzystische Ovar-Syndrom (PCOS) verursacht werden, eignet.

Das Polyzystische Ovar-Syndrom (PCOS) ist mit einer Prävalenz von 5 bis 10% eine der häufigsten Erkrankungen junger Frauen. Symptome dieser Erkrankung sind neben polyzystischen Ovarien mit fortschreitender Erkrankung Oligo- und Amenhorröe, Androgenisierungserscheinungen, wie Hirsutismus, Akne, Alopezie und/oder Adipositas. Als weitere Folgen dieser Erkrankung wird eine zunehmende Insulinresistenz und damit die Gefahr einer Erkrankung an Typ II Diabetes mellitus, eine ovulatorische Dysfunktion bis hin zur Unfruchtbarkeit, ein stark erhöhtes Risiko zur Erkrankung an Endometrium-Karzinom, Endometrium-Hyperplasie, Eierstock-Krebs und/oder Brustkrebs sowie an schwerwiegenden kardiovaskulären Erkrankungen, Insbesondere ein stark erhöhtes Risiko für Herzinfarkt beobachtet.

Zur Behandlung der durch PCOS verursachten krankhaften Störungen wird nicht nur die Bekämpfung der Fettsucht und eine drastische Gewichtsreduzierung angestrebt, sondern auch die Einnahme von östrogenhaltigen Kontrazeptiva und/oder Insulinsensitizern, wie Metformin verordnet.

Es ist aber auch bekannt, dass zumindest bei einer langfristigen Einnahme von östrogenhaltigen Kontrazeptiva die Gefahr von kardiovaskulären Erkrankungen und/oder Brustkrebs nicht völlig auszuschließen ist.

Es besteht daher ein Bedarf, ein Arzneimittel zur Verfügung zu stellen, dass zur Prophylaxe gegen oder zur Therapierung von durch das Polyzystische Ovar-Syndrom verursachte krankhafte Störungen und Spätfolgen eingesetzt werden kann, ohne das Risiko für kardiovaskuläre Erkrankungen und/oder Krebserkrankungen zu erhöhen.

Die Aufgabe wird erfindungsgemäß durch das zur Verfügung stellen eines Arzneimittels gelöst, dessen Wirkstoffkombination aus wenigstens einem kontrazeptiv wirkenden Gestagen als einzige Hormonkomponente und wenigstens einem Insulinsensitizer besteht.

Vorzugsweise enthält das erfindungsgemäße Arzneimittel als einzige hormonale Komponente wenigstens ein kontrazeptiv wirkendes Gestagen ausgewählt aus der Gruppe bestehend aus Chlormadinonacetat, 3-β-OH-Chlormadinonacetat (3β-Hydroxy-6-chlor-17α-acetoxy-4,6-pregnadien-20-on), 3-α-Hydroxy-Chlormadinonacetat (3α-Hydroxy-17α-acetoxy-4,6-pregnadien-20-on), 3α-Hydroxy-17α-acetoxy-5β-pregnan-20-on, 3β-Hydroxy-17α-acetoxy-5β-pregnan-20-on, 3-α-Acetoxy-chlormadinonacetat, 3-β-Acetoxy-chlonnadinonacetat, Dienogest, Drospirenon, Cyproteron-Acetat und Mischungen von wenigstens zwei der genannten Gestagenen, wobei die Gestagene, die nachfolgend als Komponente d), e) bzw. f) aufgeführt werden, nicht als einziges Gestagen bzw. als Gestagenmischung f) eingesetzt, sondern nur in Kombination mit einem weiteren, vorstehend aufgeführten Gestagen als einzige hormonale Komponente in der erfindungsgemäßen Wirkstoffkombination eingesetzt werden. Besonders bevorzugt werden kontrazeptiv wirkende antiandrogene Gestagene eingesetzt.

Das erfindungsgemäße Arzneimittel enthält als Insulinsensitizer-Komponente wenigstens eine Verbindung ausgewählt aus der Gruppe bestehend aus Metformin, dessen physiologisch verträglichen Salze und Glitazone. Vorzugsweise werden als Glitazone wenigstens ein Glitazon ausgewählt aus der Gruppe bestehend aus Roseglitazon und Pioglitazon und als Salz von Metformin Metforminhydrochlorid eingesetzt. Die genannten Wirkstoffe sind marktgeführte Produkte.

Das erfindungsgemäße Arzneimittel wird vorzugsweise als eine Darrelchungsform mit einer bestimmten Anzahl von Tageseinheiten zur ununterbrochenen, täglichen, oralen Verabreichung an Frauen bereitgestellt, wie dies für Kontrazeptiva üblich ist.

Jeder dieser Tages-Einheiten weist als einzige Hormonkomponente vorzugsweise einen Gestagengehalt auf, der mindestens der Ovulationshemmdosis der zum Einsatz kommenden Gestagenkomponente entspricht, vorzugsweise bis zu 150% über dieser Ovulationshemmdosis liegt. Die entsprechende Ovulationshemmdosis wird in bekannter Weise nach dem Score von "Hoogland & Skouby" bestimmt,

Vorzugsweise weist jede Tageseinheit dieselbe Menge der Gestagenkomponente auf. Darüber hinaus ist es ebenfalls besonders bevorzugt, dass Jede Tages-Einheit dieselbe Gestagenkomponente enthält. Besonders bevorzugt weist jede Tages-Einheit als einzige hormonale Komponente Chlormadinonacetet als Gestagenkomponente auf oder eine der folgenden Komponenten a) bis k), vorzugsweise mit Ausnahme jeweils der Komponente d), e) oder f) allein:
a) 3α-Hydroxy-chlormadinonacetat oder
b) 3β-Hydroxy-chlormadlnonacetat oder
c) eine Mischung von a) und b) in einem beliebigen Mischungsverhältnis oder
d) 3α-Hydroxy-17α-acetoxy-5β-pregnan-20-on oder
e) 3β-Hydroxy-17α-acetoxy-5β-pregnan-20-on oder
f) eine Mischung von d) und e) in einem beliebigen Mischungsverhältnis oder
g) eine Mischung von a) und/oder b) mit d) und/oder e) in einem belieblgen Mischungsverhältnis oder
h) eine Mischung von Chlormadinonacetat (CMA) mit a) und/oder b) in einem Mischungsverhältnis von 10 bis 90 Gew.% CMA und 90 bis 10 Gew.% von a) und/oder b), bezogen auf die Gesamtmischung, oder
i) eine Mischung von Chlormadinonacetat mit d) und/oder e) in einem Mischungsverhältnis von 10 bis 90 Gew.% Chlormadinonacetat und 90 bis 10 Gew.% von d) und/oder e), bezogen auf die Gesamtmischung, oder
j) eine Mischung von Chlormadinonacetat mit c) und f) in einem Mischungsverhältnis von 10 bis 90 Gew.% Chlormadinonacetat und 90 bis 10 Gew.% von c) und f), bezogen auf die Gesamtmischung, oder
k) eine Mischung von Chlormadinonacetat mit g) in einem Mischungsverhältnis von 10 bis 90 Gew.% CMA und 90 bis 10 Gew.% von g), bezogen auf die Gesamtmischung

Vorzugsweise enthält jede Tageseinheit dieselbe Menge der Gestagenkomponente, wobei für einen ausreichend kontrazeptiven Schutz für die Herstellung einer Tageseinheit 1 bis 10 mg, vorzugsweise jeweils wenigstens 2 mg, besonders bevorzugt jeweils 2,3,4 oder 5 mg der Gestagenkomponente Dienogest, Cyproteron-Acetat, Drospirenon, Chlormadinonacetat, eine Mischung von wenigstens 2 der genannten Gestagene oder einer der Komponenten a) bis k) mit Ausnahme jeweils der Komponente d), e) oder f) allein, verwendet werden.

Jede Tageseinheit enthält vorzugsweise dieselbe Menge der Insulinsensitizer-Komponente, wobei besonders bevorzugt jede Tageseinheit auch dieselbe Insulinsensitizer-Komponente enthält. Vorzugsweise weist eine Tageseinheit 500 bis 2000 mg, vorzugsweise 1000 bis 1500 mg Metformin, vorzugsweise als Metforminhydrochlorid, und/oder wenigstens 2 bis 45 mg eines Glitazons, besonders bevorzugt 15 bis 30 mg Pioglitazon oder besonders bevorzugt 4 bis 8 mg Roseglitazon auf.

Das erfindungsgemäße Arzneimittel eignet sich zur Prophylaxe gegen oder zur Therapierung von krankhaften Störungen, die durch das Polyzystische Ovar-Syndrom (PCOS) verursacht werden. Besonders bevorzugt eignet sich das erfindungsgemäße Arzneimittel zur Prophylaxe gegen oder zur Therapierung von Insulinresistenz, Oligo- oder Amenhorröe, Hyperandrogenemie, Tpy II Diabetes mellitus (T2DM), kardiovaskuläre Erkrankungen und/oder Prophylaxe gegen Endometrium-Karzinom, Endometrium-Hyperplasie, Eierstock-Krebs und/oder Brustkrebs neben einer vorzugsweise kontrazeptiven Wirkung. Besonders bevorzugt eignet sich das erfindungsgemäße Arzneimittel zur Prophylaxe gegen oder Therapierung von Insulinresistenz, Typ II Diabetes mellitus, androgenen Störungen, wie Hirsutismus, Akne oder Alopesie, Fettleibigkeit, weibliche Unfruchtbarkeit und/oder kardiovaskuläre Erkrankungen.

Ein weiterer Gegenstand der vorliegenden Erfindung ist daher die Verwendung der vorstehend aufgeführten Wirkstoffkombinationen bestehend aus wenigstens einer kontrazeptiv wirkenden Gestagenkomponente als einzige Hormonkomponente und wenigstens einem Insulinsensitizer zur Herstellung eines Arzneimittels zur Prophylaxe gegen und/oder zur Therapierung von vorstehend aufgeführten krankhaften Störungen, die durch das Polyzystische Ovar-Syndrom (PCOS) verursacht werden.

Dazu wird das erfindungsgemäße Arzneimittel, vorzugsweise In einer Darreichungsform zur Verfügung gestelit, die eine bestimmte Anzahl von Tageseinheiten in Form von Tabletten, die neben der erfindungsgemäßen Wirkstoffkombination gegebenenfalls noch übliche Hilfsstoffe enthalten, aufweist

Dabei kann das erfindungsgemäße Arzneimittel in Form der vorstehend beschriebenen Tageseinheiten für eine ununterbrochene Verabreichung über wenigstens 28 aufeinanderfolgende Tage zur ununterbrochenen, oralen Verabreichung bestimmt sein.

Für eine erfolgreiche Prophylaxe und/oder für eine Therapierung der durch PCOS verursachten krankhaften Störungen kann das erfindungsgemäße Arzneimittel auch in Form von Tageselnhoiten in einer solchen Anzahl zur Verfügung gestellt werden, die für eine ununterbrochene Verabreichung über mehrere Jahre, vorzugsweise bis zu 2 Jahren, besonders bevorzugt bis zu 1 Jahr, geeignet ist. Dabei ist es auch möglich, das erfindungsgemäße Arzneimittel in einer Darreichungsform zur Verfügung zu stellen, deren maximale Anzahl an Tageseinheiten für eine ununterbrochene Verabreichung an 84 Tagen oder nur an 56 Tagen geeignet Ist.

Wie bereits ausgeführt, liegt das erfindungsgemäße Arzneimittel vorzugsweise als orale Darreichungsform, ganz besonders bevorzugt in Form von Tabletten, vor. Dabei entspricht eine Tageseinheit jeweils einer Tablette. Die Tabletten werden entsprechend einen Elnnahmezyklus vorzugsweise in Blistern verpackt und vorzugsweise unter Kennzeichnung der einzunehmenden Tageseinheit als Arzneimittelpackung enthaltend wenigstens einen solchen Blister, für die verordnete, ununterbrochene Verabreichung bereitgestellt. Vorzugsweise umfasst ein Kit wenigstens 3 Blister mit jeweils 28 Tageseinheiten in einer Arzneimittelverpackung.

### Beispiele

### Beispiel 1:

### Zusammensetzung

| | Pro Tablette |
|---|---|
| Metformin • HCl | 1000 mg |
| Chlormadinonacetat | 3 mg |
| Povidon K30 | 40 mg |
| mikrokristalline Cellulose | 201 mg |
| Crospovidon | 40 mg |
| Magnesiumstearat | 8 mg |
| Hochdisperses Siliciumdioxid | 8 mg |

Povidon K 30 (Polyvinylpyrrolidon, PVP) wurde in einer ausreichenden Menge Wasser gelöst. Chlormadinonacetat (Partikelgröße 90% <50µm), mikrokristalline Cellulose, Metformin · HCl und Crospovidon wurden in einem Mischer/Granulierer (Diosna P25) 5 min gemischt und anschließend mit der wäßrigen PVP Lösung durchfeuchtet und gemischt. Die feuchte Masse wurde durch ein 3 mm Sieb getrieben und in einem Vakuumtrockenschrank getrocknet. Das trockene Granulat wurde durch ein 1,0 mm Sieb desagglomeriert, mit Magnesiumstearat und hochdispersem Siliciumdioxid gemischt und zu oblong Tabletten (9 x 21 mm) mit einem Gewicht von 1300 mg gepresst.

Die Tabletten wurden mit einem Lack auf Basis Methylhydroxypropylcellulose überzogen (z. B. Opadry YS-1-2184); Überzugsmasse 20 mg pro Tablette

28 dieser Tabletten wurden als hormonhaltige Tages-Einheiten zu einer erfindungsgemäß zum Einsatz kommenden Darreichungsform in einem Blister mit einer jeweiligen Tagesmarkierung verpackt.

### Beispiel 2:

### Zusammensetzung

| | Pro Tablette |
|---|---|
| Pioglitazon | 15 mg |
| Chlormadinonacetat | 3 mg |
| Povidon K30 | 5 mg |
| Lactose | 55 mg |
| Maisstärke | 20 mg |
| Magnesiumstearat | 1 mg |
| Hochdisperses Siliciumdioxid | 1 mg |

Povidon K 30 (Polyvinylpyrrolidon, PVP) wurde in einer ausreichenden Menge Wasser gelöst. Chlormadinonacetat (Partikelgröße 90% <50µm), Lactose, Pioglitazon und Maisstärke wurden in einem Mischer/Granulierer (Diosna P26) 5 min gemischt und anschließend mit der wäßrigen PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 7 mm Stempeln zu Tabletten mit einem Gewicht von 100 mg gepresst.

Die Tabletten wurden mit einem Lack auf Basis Methylhydroxypropylcellulose überzogen (z. B. Opadry YS-1-2184); Überzugsmasse 2 mg pro Tablette

28 dieser Tabletten wurden als hormonhaltige Tages-Einheiten zu einer erfindungsgemäß zum Einsatz kommenden Darreichungsform in einem Blister mit einer jeweiligen Tagesmarkierung verpackt.

## Patentansprüche

1. Arzneimittel, dessen Wirkstoffkombination aus wenigstens einem kontrazeptiv wirkenden Gestagen als einzige Hormonkomponente und wenigstens einem Insulinsensitizer besteht.

2. Arzneimittel nach Anspruch 1, **dadurch gekennzeichnet, dass** als Gestagen wenigstens ein kontrazeptiv wirkendes Gestagen ausgewählt aus der Gruppe bestehend aus Chlormadinonacetat, 3-β-OH-Chlormadinonacetat, 3-α**-**OH-Chlormadinonacetat, 3-α-Acetoxy-Chlormadinonacetat, 3-β-Acetoxy-Chlormadinonacetat, Dienogest, Cyproteron-Acetat, Drospirenon und Mischungen von wenigstens zwei der genannten Gestagene vorliegt.

3. Arzneimittel nach Anspruch 1 oder 2, **dadurch gekennzeichnet, dass** als Gestagenkomponente eine der folgenden Komponenten a) bis k):
a) 3α-Hydroxy-chlormadinonacetat oder
b) 3β-Hydroxy-chlormadinonacetat oder
c) eine Mischung von a) und b) in einem beliebigen Mischungsverhältnis oder
d) 3α-Hydroxy-17α-acetoxy-5β-pregnan-20-on oder
e) 3β-Hydroxy-17α-acetoxy-5β-pregnan-20-on oder
f) eine Mischung von d) und e) in einem beliebigen Mischungsverhältnis oder
g) eine Mischung von a) und/oder b) mit d) und/oder e) in einem beliebigen Mischungsverhältnis oder
h) eine Mischung von Chlormadinonacetat (CMA) mit a) und/oder b) in einem Mischungsverhältnis von 10 bis 90 Gew.% CMA und 90 bis 10 Gew.% von a) und/oder b), bezogen auf die Gesamtmischung, oder
i) eine Mischung von Chlormadinonacetat mit d) und/oder e) in einem Mischungsverhältnis von 10 bis 90 Gew.% Chlormadinonacetat und 90 bis 10 Gew.% von d) und/oder e), bezogen auf die Gesamtmischung, oder
j) eine Mischung von Chlormadinonacetat mit c) und f) in einem Mischungsverhältnis von 10 bis 90 Gew.% Chlormadinonacetat und 90 bis 10 Gew.% von c) und f), bezogen auf die Gesamtmischung, oder
k) eine Mischung von Chlormadinonacetat mit g) in einem Mischungsverhältnis von 10 bis 90 Gew.% CMA und 90 bis 10 Gew.% von g), bezogen auf die Gesamtmischung,
mit Ausnahme jeweils der Komponente d), e) oder f) allein, vorliegt.

4. Arzneimittel nach einem der Ansprüche 1 bis 3, **dadurch gekennzeichnet, dass** als Insulinsensitizer wenigstens eine Verbindung ausgewählt aus der Gruppe bestehend aus Metformin, dessen physiologisch verträgliche Salze und Glitazone vorliegt.

5. Arzneimittel nach einem der Ansprüche 1 bis 4, **dadurch gekennzeichnet, dass** als Glitazone wenigstens ein Glitazon ausgewählt aus der Gruppe bestehend aus Roseglitazon und Pioglitazon und als Salz des Metformins Metforminhydrochlorid vorliegt.

6. Arzneimittel nach einem der Ansprüche 1 bis 5, **dadurch gekennzeichnet, dass** das Arzneimittel als eine Darreichungsform mit einer bestimmten Anzahl von Tageseinheiten bestimmt zur ununterbrochenen, täglichen, oralen Verabreichung an Frauen vorliegt.

7. Arzneimittel nach Anspruch 6, **dadurch gekennzeichnet, dass** jede Tageseinheit als einzige Hormonkomponente einen Gestagen-Gehalt aufweist, der mindestens der Ovulationshemmdosis des Gestagens entspricht, vorzugsweise bis zu 150% über der Ovulationshemmdosis des Gestagens liegt.

8. Arzneimittel nach Anspruch.7, **dadurch gekennzeichnet, dass** jede Tageseinheit dieselbe Menge der Gestagenkomponente aufweist.

9. Arzneimittel nach einem der Ansprüche 6 bis 8, **dadurch gekennzeichnet, dass** jede Tageseinheit dasselbe Gestagen als Gestagenkomponente aufweist.

10. Arzneimittel nach einem der Ansprüche 6 bis 9, **dadurch gekennzeichnet, dass** eine Tageseinheit Chlormadionacetat in einer Menge von 1,5 bis 10 mg, vorzugsweise von jeweils 2, 3, 4 oder 5 mg, enthält.

11. Arzneimittel nach einem der Ansprüche 6 bis 10, **dadurch gekennzeichnet, dass** jede Tageseinheit einer Darreichungsform Chlormadionacetat jeweils einheitlich in einer Menge von 2, 3, 4, oder 5 mg enthält.

12. Arzneimittel nach einem der Ansprüche 6 bis 11, **dadurch gekennzeichnet, dass** jede Tageseinheit dieselbe Menge der insulinsensitizer-Komponente enthält.

13. Arzneimittel nach Anspruch 12, **dadurch gekennzeichnet, dass** jede Tageseinheit Metformin in einer Menge von 500 bis 2000 mg und/oder wenigstens ein Glitazon in einer Menge von 2 bis 30 mg enthält.

14. Arzneimittel nach einem der Ansprüche 6 bis13, **dadurch gekennzeichnet, dass** jede Tageseinheit dieselbe Insulinsensitizer-Komponente enthält.

15. Arzneimittel nach einem der Ansprüche 6 bis14, **dadurch gekennzeichnet, dass** die Darreichungsform wenigstens 28 Tageseinheiten aufweist.

16. Arzneimittel nach Anspruch 15, **dadurch gekennzeichnet, dass** die maximale Anzahl an Tageseinheiten einer Darreichungsform einer ununterbrochenen Verabreichung für mehrere Jahre, vorzugsweise für 2 Jahre, besonders bevorzugt für 1 Jahr entspricht.

17. Arzneimittel nach Anspruch 15, **dadurch gekennzeichnet, dass** die maximale Anzahl an Tageseinheiten einer Darreichungsform einer ununterbrochenen Verabreichung für 84 Tage entspricht.

18. Verwendung einer Wirkstoffkombination nach einem der Ansprüche 1 bis 5 zur Herstellung einer pharmazeutischen Darreichungsform nach einem der Ansprüche 6 bis 17 zur Prophylaxe gegen oder zur Therapierung von durch das Polyzystische Ovar-Syndrom (PCOS) verursachten krankhaften Störungen.

19. Verwendung nach Anspruch 18 zur Prophylaxe gegen oder zur Therapierung von Insulinresistenz, Oligo- oder Amenhorröe, Hyperandrogenaernie, Typ II Diabetes mellitus (T2 DM), kardiovaskulären Erkrankungen, Endometrium-Hyperplasie, Prophylaxe gegen Endometrium-Karzinom, Mamma-Karzinom und/oder Eierstock-Krebs.

20. Verwendung nach einem der Ansprüche 18 oder 19 zur gleichzeitigen Kontrazeption.

21. Verwendung nach einem der Ansprüche 18 oder 19 zur Prophylaxe gegen oder zur Therapierung von weiblicher Unfruchtbarkeit.

22. Verwendung nach einem der Ansprüche 18 oder 19 zur Prophylaxe gegen oder zur Therapierung von androgenen Störungen, wie Hirsutismus, Akne oder Alopezie.

23. Verwendung nach Anspruch 18 oder 19 zur Prophylaxe gegen
Insulinresistenz, Fettleibigkeit, T2DM, kardiovaskulären Erkrankungen, Endometrium-Hyperplasie, Endometrium-Karzinom, Mamma-Karzinom und/oder Eierstock-Krebs.
